# FASCICULE DE BREVET EUROPEEN

(11) **EP 0 913 361 B1**
(45) Date de publication et mention de la délivrance du brevet: **25.02.2004**
(21) Numéro de dépôt: 98402538.7
(22) Date de dépôt: 13.10.1998
(51) Int. Cl.: C02F 3/08

(54) **Filtre bactérien rotatif pour traitement aérobie**
Rotierender aerob arbeitender Biofilter
Rotating bio-filter for aerobic treatment

(30) Priorité: 16.10.1997 FR 9713143
(43) Date de publication de la demande: 06.05.1999
(73) Titulaire: Egretier, Jean-Michel, 11100 Narbonne (FR)
(72) Inventeur: Egretier, Jean-Michel, 11100 Narbonne (FR)

(56) Documents cités:
- WO-A-86/05770
- WO-A-93/22244
- CH-A- 662 805
- US-A- 3 389 798
- PATENT ABSTRACTS OF JAPAN vol. 011, no. 206 (C-433), 3 juillet 1987 -& JP 62 030600 A (TAKAHIKO ITABASHI), 9 février 1987

## Description

L'invention concerne un filtre bactérien rotatif aérobie du genre comportant un cylindre rotatif pourvu :
- à la périphérie, d'une grille à mailles fines, apte à laisser passer l'effluent à épurer ;
- intérieurement, d'un lit bactérien apte à être partiellement immergé dans ledit effluent contenu dans une cuve ;
- radialement, des cloisons aptes à compartimenter le volume intérieur dudit cylindre contenant ledit lit bactérien afin que chaque compartiment, lors de la rotation dudit filtre, soit alternativement immergé dans l'effluent à épurer ou placé à l'extérieur de celui-ci.

Un filtre bactérien connu du genre en question est décrit dans le brevet WO-8605770. Il peut être partiellement immergé dans l'eau à traiter contenue dans une cuve. Ce rotor biologique est formé de différents compartiments qui sont séparés par des supports et des murs intermédiaires. Dans les compartiments se trouvent des supports qui peuvent être réalisés en plastique ou en liège. A la périphérie, le rotor contient un manteau qui laisse passer l'eau. Après avoir passé le filtre, l'eau s'écoule vers un tunnel central d'où elle peut être évacuée.

L'invention vise à réaliser un dispositif simple, directement adaptable sur un filtre bactérien rotatif d'un type classique, destiné à :
- renouveler l'air du lit bactérien à chaque rotation et ce en quasi-totalité ;
- évacuer au fur et à mesure l'effluent directement au contact du lit bactérien à l'extérieur de la cuve.

Elle consiste donc à cet effet à réaliser :
- sur les cloisons, le long de leurs extrémités radiales, des rebords aptes, avec lesdites cloisons, à récupérer l'effluent directement au contact des parois du lit bactérien lorsque ce dernier quitte l'effluent dans lequel il était immergé et à canaliser l'effluent ainsi récupérévers la partie centrale du filtre bactérien lorsque chaque cloison passe au-dessus de l'axe dudit filtre ;
- sur le filtre bactérien, dans sa partie centrale, à l'extérieur du cylindre, un volume pourvu d'un réceptacle apte à récupérer ledit effluent canalisé, pourvu d'une ouverture basse (11) apte à évacuer, à l'extérieur de la cuve (6), l'effluent récupéré et une ouverture haute (12) apte à recycler à l'intérieur de la cuve (6), l'effluent récupéré; et en ce que l'extrémité de l 'axe (8) est soutenue par un palier (18).

Les caractéristiques et les avantages de l'invention vont apparaître plus clairement à la lecture de la description détaillée qui suit d'au moins un mode de réalisation préféré de celle-ci donné à titre d'exemple non limitatif et représenté aux dessins annexés.

Sur ces dessins :

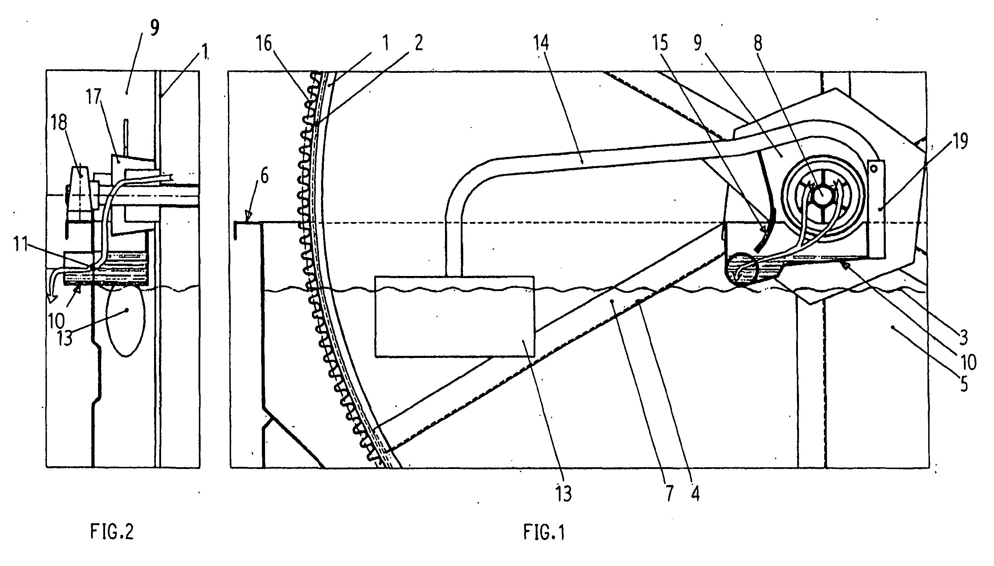

- la figure 1 est une vue en coupe transversale partielle de l'ensemble selon l'invention lorsque le niveau de l'effluent se trouve au-dessus du seuil minimum :

- la figure 2 est une vue en coupe longitudinale partielle de l'extrémité du cylindre et du réceptacle associé dans la configuration de la figure 1 ;

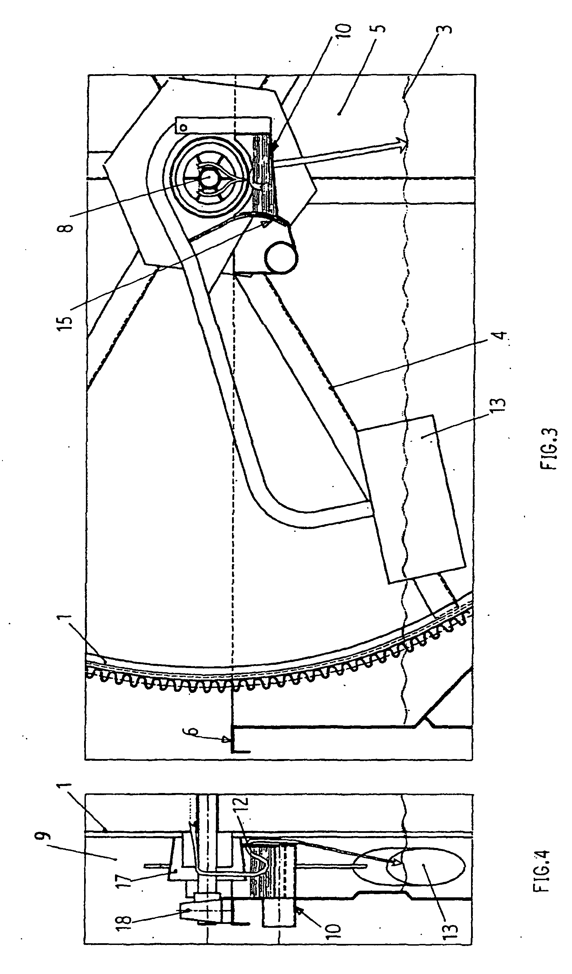

- la figure 3 est une vue en coupe transversale partielle de l'ensemble en question lorsque le niveau de l'effluent se trouve à la hauteur du seuil minimum ;
- la figure 4 est une vue en coupe longitudinale partielle de l'extrémité du cylindre et du réceptacle associé dans la configuration de la figure 3.

L'ensemble représenté aux figures comporte un cylindre rotatif (1) pourvu, à la périphérie, d'une grille (2), à mailles fines, apte à laisser passer l'effluent (3) à épurer et, intérieurement, d'un lit bactérien (5) apte à être immergé dans ledit effluent contenu dans une cuve (6).

Ledit cylindre comporte, radialement, des cloisons (4) aptes à compartimenter le volume intérieur dudit cylindre contenant ledit lit bactérien.

Le nombre de cloisons est défini pour que, le cylindre contenant le lit bactérien n'étant que partiellement immergé, chaque compartiment, lors de la rotation du filtre, est alternativement immergé dans l'effluent à épurer ou placé à l'extérieur de celui-ci.

Les cloisons (4) comportent, le long de leurs extrémités radiales, des rebords (7) aptes, avec lesdites cloisons, à récupérer l'effluent directement au contact des parois du lit bactérien lorsque ce dernier quitte l'effluent dans lequel il était immergé et à canaliser l'effluent ainsi récupéré vers la partie centrale du filtre bactérien lorsque chaque cloison (4) passe au-dessus de l'axe (8) dudit filtre.'

Le filtre bactérien comporte, dans sa partie centrale, à l'extérieur du cylindre (1), un volume (9) pourvu d'un réceptacle (10) apte à récupérer ledit film d'effluent.

Ledit réceptacle (10) comporte :
- une ouverture basse (11) apte à évacuer, à l'extérieur de la cuve (6), l'effluent récupéré ;
- une ouverture haute (12) apte à recycler, à l'intérieur de la cuve (6), l'effluent récupéré.

L'ensemble représenté comporte également, à l'extérieur du cylindre (1), un flotteur (13), au contact de l'effluent (3) contenu dans la cuve (6), relié à au volume (9) au moyen d'un bras de liaison (14), articulé, pourvu d'une paroi (15) apte à obturer l'ouverture basse (11) du réceptacle (10) lorsque le niveau de l'effluent (3) de la cuve (6) baisse au-dessous d'un seuil bien déterminé rendant ainsi opérationnelle l'ouverture haute (12) dudit réceptacle (10).

Ledit lit bactérien peut être avantageusement réalisé au moyen d'un support de bactéries en liège (broyé de préférence).

Le cylindre (1) comporte une crémaillère d'entraînement (16) associée à un pignon solidaire de l'axe d'un moto-réducteur.

l'effluent récupéré en bout d'axe (8) passe par l'élément (17) avant de se déverser dans le réceptacle (10).

L'extrémité de l'axe (8) est soutenue par un palier (18).

Le bras (14) est relié, de manière articulée, au support (19) situé dans le volume (9).

Bien entendu, l'invention n'est pas limitée aux modes de réalisation décrits et représentés pour lesquels on pourra prévoir d'autres variantes en particulier dans :
- la nature et la forme des cloisons (4,7) de séparation des compartiments contenant le lit bactérien (5) et de récupération du film d'effluent directement au contact dudit lit bactérien ;
- la nature et la forme du réceptacle (10) et de l'élément (17) ;
- la forme des ouvertures (11,12) ;
- le type de flotteur (13) et la forme de la paroi d'obturation (15) ;
- la nature du lit bactérien.

## Revendications

1. Filtre bactérien rotatif aérobie du genre comportant un cylindre rotatif (1) pourvu :
- à la périphérie, d'une grille (2), à mailles fines, apte à laisser passer l'effluent (3) à épurer ;
- intérieurement, d'un lit bactérien (5) apte à être partiellement immergé dans ledit effluent contenu dans une cuve (6) ;
- radialement, des cloisons (4) aptes à compartimenter le volume intérieur dudit cylindre contenant ledit lit bactérien afin que chaque compartiment, lors de la rotation dudit filtre, soit alternativement immergé dans l'effluent à épurer ou placé à l'extérieur de celui-ci ;
**caractérisé en ce que** les cloisons (4) comportent, le long de leurs extrémités radiales, des rebords (7) aptes, avec lesdites cloisons, à récupérer l'effluent directement au contact des parois du lit bactérien lorsque ce dernier quitte l'effluent dans lequel il était immergé et à canaliser l'effluent ainsi récupérévers la partie centrale du filtre bactérien lorsque chaque cloison (4) passe au-dessus de l'axe (8) dudit filtre ;
**en ce que** le filtre bactérien comporte, dans sa partie centrale, à l'extérieur du cylindre (1), un volume (9) pourvu d'un réceptacle (10), apte à récupérer l'effluent ainsi canalisé, pourvu d'une ouverture basse (11) apte à évacuer, à l'extérieur de la cuve (6), l'effluent récupéré et une ouverture haute (12) apte à recycler, à l'intérieur de la cuve (6), l'effluent récupéré ;
et **en ce que** l'extrémité de l'axe (8) est soutenue par un palier (18).

2. Filtre bactérien, selon la revendications 1, **caractérisé en ce qu'**il comporte, à l'extérieur du cylindre (1), un flotteur (13), au contact de l'effluent (3) contenu dans la cuve (6), relié au volume (9) au moyen d'un bras de liaison (14), articulé, pourvu d'une paroi (15) apte à obturer l'ouverture basse (11) du réceptacle (10) lorsque le niveau de l'effluent (3) de la cuve (6) baisse au-dessous d'un seuil bien déterminé rendant ainsi opérationnelle l'ouverture haute (12) dudit réceptacle (10).

3. Filtre bactérien, selon la revendication 1, **caractérisé en ce que** le lit bactérien (5) comporte un support de bactéries en liège broyé.

## Claims

1. Rotating aerobic bacterial filter of the kind including a rotary cylinder (1) provided with :
- on the periphery, a fine-meshed grid (2) adapted to let the effluent (3) to be purified pass;
- inside, a contact bed (5) adapted to be partly submerged in said effluent contained in a tank (6);
- radially, walls (4) adapted to partitioning the internal volume of said cylinder containing said contact bed in order that each compartment, when said filter rotates, is alternatively submerged within the effluent to be purified or located outside it;
**characterized in that** the walls (4) include, along their radial ends, edges (7) adapted, with said walls, to recover the effluent which is directly contacting walls of the contact bed when this latter is leaving the effluent in which it was submerged and to canalize the effluent so recovered towards the central part of the bacterial filter when each wall (4) passes above the shaft (8) of said filter,
**in that** the bacterial filter includes at its central part, outside the cylinder (1), a chamber (9) provided with a receptacle (10) adapted to recover the effluent so canalized, provided with a low opening (11) adapted to evacuate outside the tank (6) the effluent recovered and a high opening (12) adapted to recycle, inside the tank (6) the effluent recovered;
and **in that** the end of the shaft (8) is supported on a bearing (18).

2. Bacterial filter, according to claim 1, **characterized in that** it includes, outside the cylinder (1) a float (13) in contact with the effluent (3) contained in the tank (5) linked to the chamber (9) by means of a hinged linking arm (14), provided with a wall (15) adapted to close the low opening (11) of the receptacle (10) when the level of the effluent (3) of the tank (6) decreases below a well-determined threshold making that the high opening (12) of said receptacle (10) becomes operative.

3. Bacterial filter, according to claim 1, **characterized in that** the contact bed (5) includes a bacteria support of crushed cork.

## Patentansprüche

1. Rotationsfilter für aerobe Bakterien, bestehend aus einem Rotationszylinder (1), der :
- außen ein feinmaschiges Gitter (2) aufweist, durch das das zu reinigende Medium (3) einströmen kann, und
- innen ein Bakterienbett (5) aufweist, das teilweise in das erwähnte, in einem Gärbehälter (6) enthaltene Medium eingetaucht werden kann, und
- radial angeordnet Trennwände (4) aufweist, die das Innenvolumen des Zylinders mit dem Bakterienbett derart unterteilen, dass jeder Abschnitt bei der Rotation des Filters abwechselnd in das zu reinigende Medium eingetaucht oder außerhalb davon positioniert wird,
**dadurch gekennzeichnet, dass** die Trennwände (4) entlang ihrer radialen Endstücke Kanten (7) aufweisen, die zusammen mit den Trennwänden dazu geeignet sind, das sich direkt an den Wandungen des Bakterienbetts befindliche Medium abzustreifen, sobald das Bakterienbett das Medium, in das es eingetaucht war, aufnimmt, und um das so abgestreifte Medium zur Mitte des Bakterienfilters zu leiten, wenn jede Trennwand (4) über die Achse (8) des erwähnten Filters läuft;
und dass der Bakterienfilter in seiner Mitte und außerhalb des Zylinders (1) einen Bereich (9) mit einem Behälter (10) aufweist, in dem das derart abgeleitete Medium aufgenommen wird, der über eine untere Öffnung (11) verfügt, über die das aufgenommene Medium aus dem Gärbehälter (6) ausgeleitet werden kann, und der über eine obere Öffnung (12) verfügt, über die das aufgenommene Medium in den Gärbehälter (6) eingeleitet werden kann;
und dadurch, dass das Ende der Achse (8) durch ein Lager (18) abgestützt wird.

2. Bakterienfilter nach dem Patentanspruch 1, **dadurch gekennzeichnet, dass** er auf der Außenseite des Zylinders (1) einen Schwimmer (13) aufweist, der in Kontakt zu dem im Gärbehälter (6) befindlichen Medium (3) steht, und mit dem Bereich (9) über einen Verbindungsgelenkarm (14) mit einer Wandung (15) verbunden ist, mit dem die untere Öffnung (11) des Behälters (10) verschlossen werden kann, wenn der Stand des Mediums (3) im Gärbehälter (6) unter einen entsprechend definierten Schwellwert absinkt, damit die obere Öffnung (12) in Betrieb bleibt.

3. Bakterienfilter nach dem Patentanspruch 1, **dadurch gekennzeichnet, dass** das Bakterienbett (5) eine Bakterienauflage aus zerstückelten Korkstücken aufweist.
